Europäisches Patentamt

European Patent Office

Office européen des brevets

(11) Publication number: **0 241 435 B1**

# EUROPEAN PATENT SPECIFICATION

(45) Date of publication of patent specification: **02.12.92**   (51) Int. Cl.5: **C12N 15/63**, C12N 1/16

(21) Application number: **87830114.2**

(22) Date of filing: **25.03.87**

The file contains technical information submitted after the application was filed and not included in this specification

(54) **Vectors for the cloning and expression of heterologous genes in yeast and the yeast strains transformed by said vectors.**

(30) Priority: **27.03.86 IT 4783086**

(43) Date of publication of application:
**14.10.87 Bulletin 87/42**

(45) Publication of the grant of the patent:
**02.12.92 Bulletin 92/49**

(84) Designated Contracting States:
**AT BE CH DE ES FR GB GR LI LU NL SE**

(56) References cited:
**EP-A- 0 095 986**
**EP-A- 0 096 430**
**EP-A- 0 096 910**

**JOURNAL OF BACTERIOLOGY, vol. 145, no. 1, January 1981, pages 382-390; N. GUNGE et al.: "Isolation and characterization of linear deoxyribonucleic acid plasmids from Kluyveromyces lactis and the plasmid-associated killer character"**

(73) Proprietor: **UNIVERSITA' DEGLI STUDI DI ROMA**
**"LA SAPIENZA" P.le Aldo Moro 5**
**I-00185 Rome(IT)**

Proprietor: **CONSIGLIO NAZIONALE DELLE RICERCHE**
**Piazzale Aldo Moro, 7**
**I-00185 Roma(IT)**

Proprietor: **CENTRE NATIONAL DE LA RE-CHERCHE SCIENTIFIOUE (CNRS)**
**15, Ouai Anatole France**
**F-75700 Paris Cedex 07(FR)**

(72) Inventor: **Claudio, Falcone**
**Via le Bruno Buozzi 107**
**I-00197 Rome(IT)**
Inventor: **Hiroshi, Fukuhara**
**Batiment 7 160 Avenue du General Leclerc**
**F-91190 Gif sur Yvette(FR)**
Inventor: **Laura, Frontali**
**Via Adige 30**
**I-00198(IT)**

**NUCLEIC ACIDS RESEARCH, vol. 14, no. 11, 1986, pages 4471-4481, IRL Press Ltd, Oxford, GB; K.J. CHEN et al.: "Sequence organization of the circular plasmid pKD1 from the yeast Kluyveromyces drosophilarum"**

**PLASMID 15, 1986, pages 248-252, Academic Press Inc., C. FALCONE et al.: "Analysis of a 1.6-mum circular plasmid from the yeast Kluyceromyces drosophilarum: structure and molecular dimorphism"**

**NUCLEIC ACIDS RESEARCH, vol. 12, no. 19, October 1984, pages 7581-7597, IRL Press Ltd, Cambridge, GB; F. HISHINUMA et al.: "Cloning and nucleotide of the linear DNA killer plasmids from yeast"**

74 Representative: **de Simone, Domenico et al Ing. Barzanò & Zanardo Roma S.p.A. Via Piemonte 26 I-00187 Roma(IT)**

**Description**

The present invention concerns some cloning and expression vectors for heterologous genes in yeasts as well as the yeast strains transformed by said vectors.

In more detail, the present invention concerns vectors for the transformation of yeasts, in which vectors the entire nucleotide sequence of the pKD1 plasmid of Kluyveromyces drosophilarum or a part of the same is present. Moreover, the present invention concerns specifically yeasts of the Kluyveromyces genus transformed by said vectors.

As is well known, the transformation of yeasts up to the present time has been limited to certain species because of the poor availability of the yeast plasmids that are required for the construction of transformation vectors (Gunge, N., Ann. Rev. Microbiol. 37, 253-276 (1983); Toh-e, A., Tada, S. and Oshima, Y., J. Bacteriol. 151, 1380-1390 (1982); Toh-e, A., Araki, H., Utatsu, I., and Oshima, Y., J. Gen. Microbiol. 130, 2527-2534 (1984)).

The 2μ plasmid isolated from Saccharomyces cerevisiae has been, up to the present time, the only plasmid successfully employed for the transformation of said S. cerevisiae. However, vectors derived from the 2μ plasmid have shown a low transformation efficiency and/or a limited ability to replicate stably in yeasts other than Saccharomyces cerevisiae.

Accordingly, it was quite evident that plasmids for the construction of vectors suitable for the transformation of other yeasts were needed, especially for yeasts of industrial importance, including, for example, those belonging to the genus Kluyveromyces.

However, all attempts to date to satisfy this necessity have not been very effective.

More specifically, two vectors have been used for the transformation of Kluyveromyces lactis: one contained a chromosomal DNA segment of K. lactis (Das, S. and Hollenberg, C.P., Current Genetics 6 123-128 (1982), and the other was derived from the linear plasmid pGK1, also isolated from K. lactis (L. de Louvencourt, H. Fukuhara, H. Heslot, M. Wesolowski, French patent application No. 8209564 of ELF Biorecherche; L. de Louvencourt et al., J. Bacteriol. 154, 737-742 (1982)).

It is important to point out that, although both of the vectors mentioned above contain nucleotide sequences of K. lactis that allow their replication in this yeast, the efficiency and/or the stability with which transformed clones are obtained is quite low.

Accordingly, an object of the present invention is the production of cloning and expression vectors for yeasts, in particular for the genus Kluyveromyces, which have improved transformation efficiency and which show a higher stability in the transformed cells.

Now it has been surprisingly found, according to the present invention, that this object can be attained by using as an essential part of the transformation vectors, a plasmid discovered in a strain of the yeast Kluyveromyces drosophilarum, in the course of a research project on new plasmids carried out by C. Falcone, L. Frontali and H. Fukuhara (the inventors designated for the present patent application).

The plasmid dealt with herein consists of a circular duplex DNA molecule of 1.6 micron circumference, which was found in the UCD 51-130 Kluyveromyces drosophilarum strain (U.C.D. Collection, University of California, Davis, CA 95616).

Said plasmid, which is called pKD1, is present in the cells in a high copy number (70-100 per cell) and it is easily separable from the chromosomal DNA by employing standard procedures.

DNA hybridization experiments confirmed the substantial difference in primary structure between this plasmid and the 2μ and pGK1 plasmid.

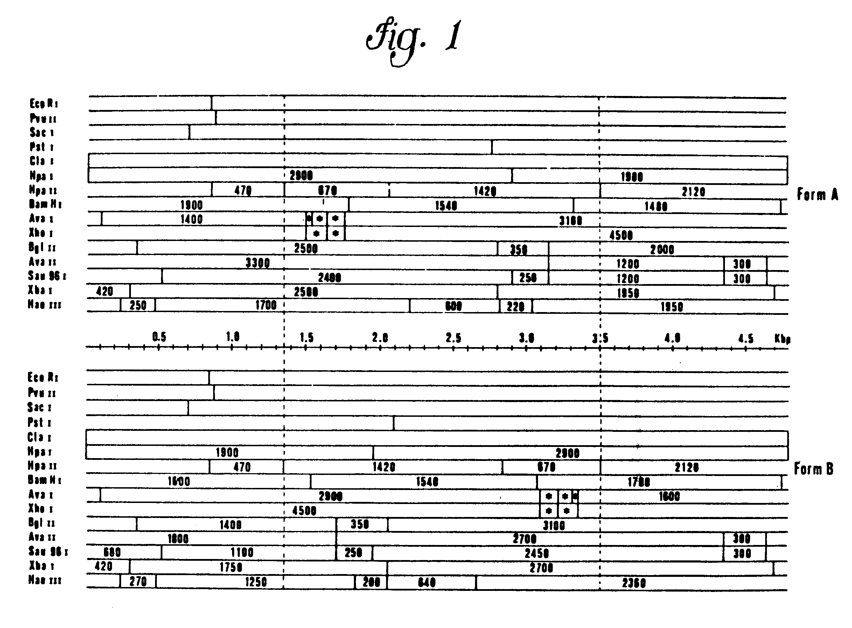

The plasmid exists inside the same cell in two interchangeable molecular forms called A and B, which are present in equal amounts and differ from one another by the orientation of a central segment of 2.15 kilobases that, in Figure 1 enclosed herein, is comprised between two vertical dashed lines. Figure 1 shows the localization of the sites for numerous restriction enzymes (such location referring to the A and B forms of pKD1 when drawn as linear molecules), such enzymes being indicated in the left margin of said figure. The scale shown in the horizontal direction points out the distance in kilobases (kb) between these sites on the plasmid. Numbers shown inside the segments indicate their respective lengths in base pairs. The asterisks point out those fragments whose localization is uncertain.

The analysis of the sequence showed the presence in this plasmid of a DNA segment of 346 base pairs which was found again, identical in sequence but with inverted orientation, at a distance of 2136 nucleotides.

This inverted and repeated (TR) sequence is likely to be fundamental in the mechanism of interconversion between the A and B forms of pKD1, in analogy with what has been observed with the 2μ plasmid of S. cerevisaiae.

From the nucleotide sequence of the plasmid pKD1 the presence of three possible genes, called A, B and C, was deduced (with respective lengths of 1341, 1245 and 636 nucleotides); these genes, based on sequence homologies and preliminary studies on their functions, seem to be analogous, respectively, to the FLP, Rep 1 and Rep 2 genes that are present in the $2\mu$ plasmid (Broach, J.R., Cell 28, 203-204 (1982)).

The pKD1 plasmid can be advantageously employed for the construction of cloning and expression vectors in yeasts, primarily because its circular form allows simple manipulations. Moreover, this plasmid contains numerous unique sites for restriction enzymes, such as EcoRI, ClaI and PstI, and this feature is also particularly advantageous for the insertion of heterologous genes and for the construction of recombinant vectors.

The presence of the pKD1 plasmid and of the vectors derived from the same in the cell at a high copy number is a further advantage, because it allows an amplification of the inserted heterologous gene.

Moreover, it is advantageous that neither the integrity nor the continuity of the sequence of the pKD1 plasmid is needed for the construction of vectors according to the present invention. Indeed, it is possible to insert the gene to be cloned into one of the unique restriction sites so interrupting the pKD1 sequence, or to use just a segment containing the replication origin of this plasmid for the objects of the present invention.

The specific object of the present invention consists in the construction of cloning and expression vectors for heterologous genes in yeasts, such vectors containing at least: all or part of the DNA of the pKD1 plasmid (isolated from Kluyveromyces drosophilarum) and a DNA segment bearing any heterologous gene, including the sequences that insure the expression of said gene.

When the entire DNA of the pKD1 plasmid is employed, it is preferable that the gene to be cloned be inserted into one of the unique restriction sites, in particular into the PstI site.

Among the genes that are most suitable for cloning, the URA3 gene of S. cerevisiae is in particular worth considering, preferably as a part of the YIP5 plasmid, which consists of the sequence of the pBR322 plasmid of Escherichia coli and, in addition, of the sequence of this URA3 gene.

In another preferred form of the present invention, the vector contains the 1.7 Kb segment of DNA of the pKD1 plasmid obtained with the enzyme BamHI, inserted into the unique BamHI site of the YIP5 plasmid.

The present invention also relates to yeasts transformed by the above-mentioned vectors and, particularly but not exclusively, to the strains of the genus Kluyveromyces and more specifically to the genus Kluyveromyces lactis.

It is interesting to note that yeasts transformed according to the present invention can be advantageously exploited for the production of the protein coded by the heterologous gene inserted into the vector. Such yeasts can be, for example, of remarkable importance in the foodstuff field (the production of amylase, rennin, pectinase and so on) or in the pharmaceutical field (insulin, interferon and so on).

In additon, such transformed yeasts can constitute in themselves integrating products for the feeding of animals or livestock (biomass).

For the sake of discussion, and for illustrative but not limitative purposes, the use of the invention for the cloning and expression of the URA3 gene of Saccharomyces cerevisiae in a uraA strain of Kluyveromyces lactis will be considered. The uraA strain is normally unable to grow in uracil-free medium. The URA3 gene that is present in vectors derived from the pKD1 plasmid was shown to be expressed in the host cell and such cells were shown to be able to grow in uracil-free mediums. This shows that the orotidine-monophosphate decarboxylade protein encoded by the URA3 gene of S. cerevisiae is effectively synthesized by K. lactis.

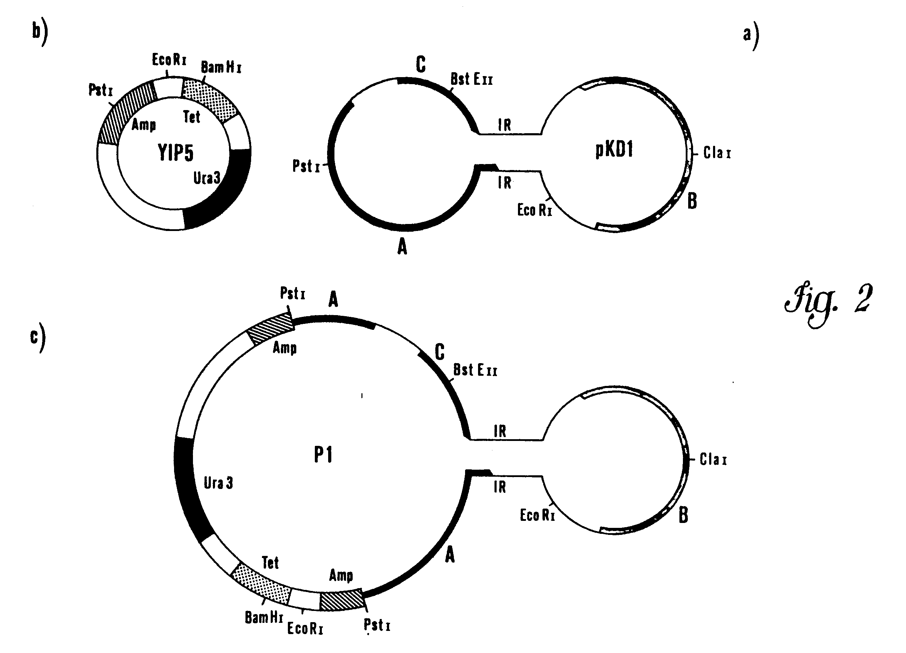

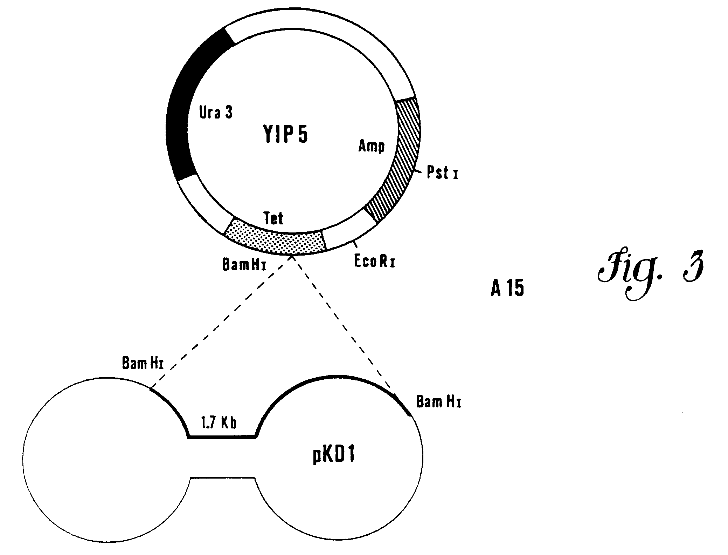

In the following discussion reference will be made to Figures 2 and 3 of the enclosed drawings wherein:

Figure 2a shows schematically the structure of the B form of the pKD1 plasmid;

Figure 2b shows the YIP5 plasmid;

Figure 2c shows the P1 vector according to the present invention; and

Figure 3 shows the A15 vector according to a further embodiment of the present invention.

## The construction of vectors derived from the pKD1 plasmid that contain the URA3 gene.

## The isolation and purification of the pKD1 plasmid

The plasmid DNA was pepared from the protoplasts obtained from 2 l of a culture of K. drosophilarum. Nucleic acids obtained after lysis of the protoplasts were resuspended in 40 ml of a solution containing 50 mM Tris-HCl, 5mM EDTA, pH 8.0. 40 g of CsCl and 4 ml of ethidium bromide (10 mg/ml in 50 mM Tris-HCl, pH 8.5) were added to the DNA-containing solution which was then centrifuged at 38,000 rpm in a Beckman 50 Ti rotor for 40 hours at 15°C. The DNA was visualized with U.V. light and the lower band, corresponding to the plasmid DNA, was recovered from the gradient and purified a second time according to the procedure described above.

## pKD1-derived vectors

Now reference will be made in a specific way to Figure 2a in which pKD1 is shown in the circular closed form with the inverted and repeated (TR) sequences paired to one another. The zones shown in black indicate the localization of the main genes present in the plasmid. In addition, the positions of the most important restriction sites for the cloning and manipulation of the plasmid, are shown.

In this figure the B form of plasmid is shown. The form differs from the B form by the orientation of the circle on which the gene B is localized, as already detailed above.

For the construction of recombinant plasmids, pKD1 was first linearized at the unique PstI site. At the same time, YIP5, which is a recombinant plasmid containing sequences of the URA3 gene of Saccharomyces cerevisiae and of the bacterial plasmid pBR322 was also linearized at the PsTI site. The structure of YIP5 is shown in Figure 2b, in which the sequences of the URA3 gene of S. cerevisiae are shown in black, and the sequences of the bacterial plasmid pBR322 are shown in white, with the genes that are responsible for the resistance to ampicillin (Amp) and tetracycline (Tet). Furthermore, the position of some unique restriction sites used for cloning are shown.

The two linearized molecules were joined at the common PstI site by DNA ligase according to a well-known standard procedure.

The plasmid obtained by this ligation thus contains the entire pKD1 sequence, the URA3 gene and the pBR322 sequence. The latter sequence was introduced with the aim of amplifying the constructed vectors in the bacterium Escherichia coli.

The ligation reaction mixture was added directly to a suspension of E.coli cells of the strain RR1, which had been made competent for transformation after treatment with calcium chloride.

The cell suspension was plated on a solid tetracycline-containing complete medium. Among the thousands of colonies obtained (which, therefore, were tetracycline-resistant) a number of colonies that were unable to grow on an ampicillin-containing medium were selected. These colonies (tetracycline-resistant and ampicillin-sensitive), which represented a fraction of about 10%, were examined individually for the nature of the plasmid they contained.

It was found that about half of such colonies contained a recombinant plasmid having the desired structure, as shown in Figure 2c, wherein the PI vector is shown. Symbols employed are the same as those adopted in Figures 2a and 2b.

With this procedure another vector was also constructed, called P3, which differs from P1 by the fact that it contains the A form of pKD1.

The A15 plasmid is another recombinant molecule derived from pKD1. The latter was digested with the restriction enzyme by BamHI and a fragment of 1700 base pairs was isolated. This fragment was then mixed with the linearized molecule of YIP5, which had ben cut at its unique BamHI site. After ligation and amplification in E.coli, as described above, a clone was selected that shows resistance to ampicillin and sentitivity to tetracycline. A recombinant plasmid was isolated from this clone, and the plasmid contained, as expected, the BamHI fragment of pKD1, the URA3 gene and the sequence of pBR322.

In Figure 3, which relates to the construction of the A15 vector, the insertion point of the 1.7 Kb BamHI fragment (in black) of pKD1 in the YIP5 plasmid is shown. Symbols adopted are the same as those employed in the preceding figures.

The vectors constructed as discussed above using pKD1, the URA3 gene and the sequences of pBR322 that allow replication in E.coli, were purified from the clones of E.coli previously described and then used for the transformation of a uraA strain of Kluyveromyces lactis.

## Transformation of Kluyveromyces lactis

Previous studies had shown that the uraA mutation of K.lactis could be complemented by the URA3 gene of S.cerevisiae (L. de Louvencourt et al., J. Bacteriol. 154, 737-742 (1982)). Therefore, a strain of K.lactis containing this mutation was used to verify the transformation with hybrid plasmids consisting of pKD1 sequences and the URA3 gene.

The strain MW98-8C (a, uraA, arg, lys, $K^+$, $R^+$), isolated in the Orsay laboratory from the CBS 2359 strain of K.lactis, was grown in liquid medium containing 2% glucose, 1% yeast extract and 1% bactopeptone, for 18 hours at 28°C in a shaker bath.

The cells, in the phase of exponential growth, were converted into protoplasts according to a standard procedure.

The protoplasts were resuspended in STC buffer (1 M sorbitol, 10 mM $CaCl_2$, 10 mM Tris-HCl, pH 7.5) at a concentration of about $10^9$ per ml. An aliquot of this suspension, usually 0.1 ml, was then mixed with 0.2-0.5 $\mu$g of the P1, P3 and A15 vectors described in the present invention and of the control plasmids.

After 10 minutes, the suspension was heated to 42°C for 2 minutes and then mixed with one volume of polyethylene glycol 4000. After a further period of 10 minutes at room temperature, the suspension was centrifuged and the spheroplasts were washed and resuspended in SOS buffer (100 ml of the latter contain: 50 ml of 2 M sorbitol, 33.5 ml of YEP medium, 0.65 ml of 1M $CaCl_2$, 135 $\mu$l of 1% uracil and 15 ml of water).

0.1 ml aliquots of the spheroplast suspension were added to 4 ml of W medium (which was kept liquid at 45°C and contained the supplements that complemented the auxotrophies other than uracil) and then poured onto Petri dishes containing solid W medium.

The growth of colonies was observed after 3-4 days incubation at 28°C.

Table 1, below, shows the number and the stability of the transformation experiments of the Kluyveromyces lactis MW98-8C strain by the vectors derived from the pKD1 plasmid.

TABLE 1

| Vector | Number of ura transformants[a] | Stability of the transformants[b] |
|--------|-------------------------------|-----------------------------------|
| P1 | 9800 | 18 |
| P3 | 6300 | 30 |
| A15 | 6700 | 3.6 |
| YIP5 | 0 | - |

a) The number of transformants is the average value obtained in 2-3 experiments in which 0.2-0.5 $\mu$g of plasmid DNA was used.
b) Stability is expressed as the percentage of ura$^+$ colonies obtained after six generations in nonselective medium.

Only those protoplasts to which a pKD1 sequence-containing plasmid was added gave rise to the formation of colonies that are therefore ura$^+$. In the case of YIP5, which does not contain any pKD1 sequences, colonies were observed only after addition of uracil to the growth medium.

DNA was extracted from some colonies transformed with pKD1-derived vectors, and the presence of a plasmid whose restriction map was identical to the map of the plasmid used for the transformation was observed.

As can be observed in the Table, the pKD1 DNA fragment contained in A15 is sufficient in itself to promote the replication of the recombinant vector. Other vectors not containing this pKD1 region are unable to transform Kluyveromyces lactis. However, the efficiency of transformation of the A15 vector and the stability of the resulting transformants are lower than those observed with the other vectors. This suggests that the A15 vector lacks some pKD1 sequences that could have an important role in plasmid maintenance.

The stability of the transformants obtained with the P1 and P3 vectors, which contain the entire pKD1 plasmid, is by far the highest observed to date in the transformation of Kluyveromyces.

This is highly advantageous for industrial applications involving yeasts transformed by these vectors.

The present invention has been discussed with particular reference to some specific aspects, but it is to be understood that modifications and changes can be introduced by those who ore skilled in this field without departing from the spirit and scope of the invention.

**Claims**

1. Cloning and expression vectors for heterologous genes in yeasts, such vehicles being characterized in that they contain at least: the entire DNA of the pKD1 plasmid (isolated from Kluyveromyces drosophilarum) or a part thereof, and a DNA segment bearing any heterologous gene, including sequences that insure the expression of said gene.

2. A vector according to the claim 1 wherein said heterologous gene is inserted into one of the unique restriction sites of said pKD1 plasmid.

3. A vector according to claim 2 wherein said unique restriction site is the PstI site.

4. A vector according to claims 1-3 wherein said heterologous gene is the URA3 gene of Saccharomyces cerevisiae.

5. A vector according to claim 4 wherein said DNA segment bearing said URA3 gene is DNA of the YIP5 plasmid.

6. A vector according to claim 5 containing the DNA fragment of 1.7 kilobases length of said pKD1 plasmid, said fragment being obtained with the enzyme BamHI, and being inserted at the unique BamHI site of said YIP5 plasmid.

7. Yeasts transformed by the vectors described in claims 1-6.

8. Yeasts according to claim 7 belonging to the genus Kluyveromyces.

9. Yeasts according to claim 8 belonging to the species Kluyveromyces lactis.

**Patentansprüche**

1. Klorierungs- und Expressivitaets-vektore in Gaerungen fuer Heterologgene dadurch gekennzeichnet, dass sie mindestens die ganze DNS der pKD1-Plasmide (isoliert aus Kluyveromyces Drosophiliarum) oder ein Teil derselben und ein irgendein Heterologgen tragendes DNS-Segment, einschliesslich Sequenzen, die die Expressivitaet des vorgenannten Gens versichern, enthalten.

2. Vektor nach Anspruch 1, bei dem das vorgenannte Heterologgen in einen von den einzelnen Restriktionssiten des vorgenannten pKD1-Plasmids eingesetzt ist.

3. Vektor nach Anspruch 2, bei dem der vorgenannte Restrictionsite der PstI-Site ist.

4. Vektor nach Anspruechen 1 bis 3, bei dem das vorgenannte Heterologgen das URA3-Gen von Saccharomyces Cerevisiae ist.

5. Vektor nach Anspruch 4, bei dem das vorgenannte, das genannte URA-Gen tragende DNS-Segment die DNS des YIP-Plasmids ist.

6. Vektor nach Anspruch 5, der ein 1,7 Kilobasen larges DNS-Fragment des vorgenannten pKD1-Plasmids enthaelt, wobei dieses Fragment mit dem BamHI-Enzym erhalten und in den einzigen BamHI-Site des vorgenannten YIP5-Plasmids eingesetzt wird.

7. Durch die in den Anspruechen 1 bis 6 beschriebenen Vektoren transformierte Gaerungen.

8. Gaerungen nach Anspruch 7, die zum Kluyveromyces-Gen zugehoeren.

9. Gaerungen nach Anspruch 8, die zum Kluyveromyces Lactis-Species zugehoeren.

**Revendications**

1. Vecteurs de clonation et d'expressivité pour gènes hétérologues dans les leveures, caracterisés en ce que ils comprendent au moins: l' ADN entier du plasmide pKD1 (isolé de Kluyveromyces drosophilarum) ou une partie de ce plasmide et un segment d'ADN portant un gène hétérologue, ayant séquences qui assurent l'expressivité dudit gène.

2. Vecteur selon la revendication 1, dans lequel ledit gène hétérologue est inseré dans un des sites de restriction uniques dudit plasmide pKD1.

3. Vecteur selon la revendication 2, dans lequel ledit site de restriction unique est le site PstI.

4. Vecteur selon le revendications 1-3, dans lequel ledit gène hétérologue est le gène URA3 de Saccharomyces cerevisiae.

**5.** Vecteur selon la revendication 4, dans lequel ledit segment d'ADN portant ledit gène URA3 est ADN du plasmide YIP5.

**6.** Vecteur selon la revendication 5, comprenant le fragment d'ADN de longueur de 1,7 kilobases dudit plasmid pKD1, ledit fragment étant obtenu par l'enzyme BamHI and étant inséré dans le site unique BamH1 dudit plasmide YIP5.

**7.** Levures transformées par le vecteurs decris dans les revendications 1-6.

**8.** Levures selon la revendication 7 appartenantes au genre Kluyveromyces.

**9.** Levures selon la revendication 8 appartenantes à l'espèce Kluyveromyces lactis.

# Fig. 1

Eco Rı
Pvu ıı
Sac ı
Pst ı
Cla ı
Hpa ı — 2900 — 1600
Hpa ıı — 470 — 870 — 1420 — 2120
Bam Hı — 1900 — 1540 — 1400 — **Form A**
Ava ı — 1400 — 3100
Xbe ı — 4500
Bgl ıı — 2500 — 350 — 2000
Ava ıı — 3300 — 1200 — 300
Sau 96 ı — 2400 — 250 — 1200 — 300
Xba ı — 420 — 2500 — 1950
Hae ııı — 250 — 1700 — 600 — 220 — 1950

0.5   1.0   1.5   2.0   2.5   3.0   3.5   4.0   4.5   Kbp

Eco Rı
Pvu ıı
Sac ı
Pst ı
Cla ı
Hpa ı — 1900 — 2900
Hpa ıı — 470 — 1420 — 870 — 2120
Bam Hı — 1600 — 1540 — 1700 — **Form B**
Ava ı — 2900 — 1600
Xbe ı — 4500
Bgl ıı — 1400 — 350 — 3100
Ava ıı — 1800 — 2700 — 300
Sau 96 ı — 680 — 1100 — 250 — 2450 — 300
Xba ı — 420 — 1750 — 2700
Hae ııı — 270 — 1250 — 200 — 640 — 2360

EP 0 241 435 B1

Fig. 2

Fig. 3